# EUROPEAN PATENT APPLICATION

(11) **EP 0 919 190 A2**
(43) Date of publication of application: **02.06.1999**
(21) Application number: 98309545.6
(22) Date of filing: 23.11.1998
(51) Int. Cl.: A61B 10/00

(54) **Biopsy instrument with continuous tissue receiving chamber**

(30) Priority: 24.11.1997 US 976705
(71) Applicant: ETHICON ENDO-SURGERY, INC., Cincinnati, OH 45242-2839 (US)
(72) Inventor: Voegele, James W., Cincinnati, Ohio 45249 (US); Erickson, Paul L., Cincinnati, Ohio 45236 (US); Privitera, Salvatore, West Chester, Ohio 45069 (US); Rhad, Edward, Fairfield, Ohio 45014 (US)
(74) Representative: Mercer, Christopher Paul

(57) **Abstract**

A surgical instrument for excising a targeted tissue mass to be biopsied from adjacent bodily tissue of a surgical patient is disclosed. The instrument has an elongated tube, a penetrating member with first and second tissue stops affixed to the distal end of the elongated tube, and a tissue cutter mounted concentrically relative to the elongated tube for movement from first to second positions for cutting captured tissue received within a tissue receiving chamber. The first and second tissue stops define the tissue receiving chamber continuously about a 360° arc at the distal end of the elongated tube for receiving the targeted tissue mass therein. Unlike conventional biopsy instruments where tissue is received within a confined lateral receiving port, a significantly larger sample size can be taken within the tissue receiving chamber. The instrument is adapted for taking sufficiently sized samples to be biopsied in a minimally invasive manner, minimizing or avoiding trauma adjacent the biopsy site.

## Description

### Background of Invention

This invention relates to an instrument for excising a targeted tissue mass. Specifically, it relates to an instrument for excising an appropriately sized tissue mass from a targeted lesion which is to be biopsied for determination of malignancy, particularly for breast cancer.

Statistics currently reveal that one in nine American women will develop breast cancer. It is the leading cause of cancer deaths in women between the ages of 40-55, and the second leading cause of cancer deaths in women overall. Unfortunately, breast cancer will only be diagnosed in about one in eight women, and one in thirty will die of this disease. Breast cancer does occur in men but it is much more uncommon.

A biopsy of the breast is often indicated if suspicious tissue is detected. Biopsy requests stem from a screening process generally performed via a physical examination to detect a palpable lesion or a mammogram for the detection of a nonpalpable lesion. Five out of six biopsies performed return benign indications.

The goal of the biopsy is the discovery and accurate diagnosis of benignity or malignancy of suspect tissue. Survival rates are significantly greater with early detection. However, as detection is attempted in earlier stages of carcinoma development, accurate sampling of the lesion becomes more difficult due to small lesion size. Ideally, a sufficiently large sample size is taken to accurately diagnosis disease state with minimal trauma (physical deformity or scarring) to the patient.

The current standard for care is a significantly invasive procedure known as "Open Excisional Biopsy". Prior to an open excisional biopsy for a nonpalpable mass, an interventional radiologist will initially take mammograms to place a guidewire which directs the surgeon to the area of excision. Mammograms are then taken to assure that the guidewire is accurately placed in or near the desired biopsy site. The guidewire has a barbed end in order maintain its position at the desired site and is fed through an elongated needle.

The general surgeon applies local or, more commonly, general anesthesia, during an open excisional biopsy. Dissection is made through the skin and subcutaneous fat and the flap is undercut and retracted. Incision length, during an open excisional biopsy generally runs between 2-5 inches and is dependent on the location of the lesion, breast characteristics, and surgeon technique. The breast parenchyma is then dissected along the guidewire until the site of the specimen is exposed. The specimen, generally spherical in shape centered about the barbed end of the guidewire, and roughly 1 inch in diameter, is manually excised.

The specimen size taken during an open excisional biopsy is adequate, however deformation (scarring and subsequent shape of the breast) and potential "seeding" of the cancerous cells is often a large concern. Accuracy is generally acceptable, however the guidewire often moves from its original position during the excisional process leading to decreased accuracy and the need for larger specimens.

New technologies are being developed to assist the early detection of breast cancer. The technologies are primarily focused on imaging of nonpalpable tissue and accessing the tissue with minimally invasive techniques for biopsy. The cost and trauma associated with open excisional biopsy is minimized with these technologies.

The two primary imaging technologies for assisting minimally invasive breast biopsy of a nonpalpable mass are ultrasound and stereotactic X-rays. Ultrasound accurately guides a hand-held core biopsy needle (CBN) to the biopsy site. Stereotactic imaging is performed with the patient lying prone on a stereotactic table and X-rays are taken at 15 degrees off-axis on each side of the breast. The X-rays are fed into a digital signal imaging system to create a 3-D reference; the location for biopsy is triangulated and coordinates are fed to the stereotactic table.

The actual specimen removal in the minimally invasive approach is performed in any one of the following ways.

TruCut® Needle Method - The "TruCut" needle method utilizes a small diameter needle which is inserted to a desired depth, actuated, and removed. The product for practicing this method has two co-axial tubular members. The outer member is retracted exposing a lateral opening in the inner member. The elastic property of the tissue causes tissue to enter the hollow core of the inner member. The outer member is then advanced forward, shearing the tissue sample which has been trapped in the inner member. Inherent disadvantages to this technique are the need for multiple insertions with questionable accuracy and extremely limited tissue sample sizes. The TruCut® needle and its method of use are described in U.S. Patent 4,958,625.

Surface to Site Core Sampling (SSCS) - The SSCS method attempts to maximize accuracy through the increase in sample size while maintaining controlled guidance. A cylindrical cutting tube is advanced through an opening in the skin. As it moves forward, it creates a continuous cylindrical core sample until it reaches the depth desired by the surgeon. Upon completion of the linear motion, the distal end of the sample is transected, and the device is withdrawn with the sample within the cutting element. The critical disadvantage to this approach is the trauma and disfigurement associated with excision of the healthy tissue superior to the site of questionable tissue.

Percutaneous Core Biopsy (PCB) - The PCB method attempts to obtain the advantages of single insertion and large sample size of the SSCS approach, while minimizing disfigurement as achieved by the TruCut® needle method. As in the case of a TruCut® needle device, the PCB has multiple co-axial tubular members. However, one of the members remains stationary, eliminating the need for multiple insertions. Samples are obtained in a controlled contiguous manner which ultimately achieves the desire for obtaining large tissue samples at the questionable site.

A recent minimally invasive approach for excising a portion of a targeted lesion is described in U.S. Patent 5,526,822. The biopsy apparatus described in this patent has an outer biopsy cannula with a penetrating member for positioning adjacent the targeted lesion. The biopsy cannula has a lateral tissue receiving port, and a vacuum is drawn through the port for pulling tissue into the port. An inner cutting cannula moves forwardly to excise the tissue received in the port. With the aid of a vacuum drawn on the inner cutting cannula, the inner cutting cannula retains the excised tissue as the inner cannula is retracted for tissue removal, and the outer biopsy cannula remains stationary. Following removal, the outer biopsy cannula can be rotated relative to a fixed housing, and multiple samples can therefore be taken circumferentially without the need for multiple insertions and withdrawals of the biopsy cannula. The biopsy apparatus and its method of use as described in the '822 patent are exemplified in the Mammotome® Breast Biopsy System.

Another device for minimally invasive biopsy is disclosed in Japanese Kokoku Patent No. Sho 57 [1982]-15892. This device has a tubular outer needle with an annular beveled cutting edge at its distal end, an inner needle with a conical distal tip and a small diameter portion occupying a length proximal to the conical tip, and a means for adjustably fixing the relative axial position between the outer and inner needle. When piercing into tissue, the conical tip protrudes from the distal end of the outer needle to create a gap between the outer diameter of the conical tip and the inner diameter of the outer needle cutting edge. Tissue is cut by the outer needle cutting edge and passes through this gap into the small diameter portion of the inner needle. After the device is inserted to the appropriate depth, the inner needle is released and withdrawn from the outer needle. The withdrawal force is intended to sever the tissue sample collected continuously about the small diameter portion of the inner needle from the surrounding tissue. Although this device is intended to collect a sample continuously about an inner tubular needle, it relies on tissue passively entering the small gap between the conical tip of the inner needle and the outer needle cutting edge. The effect of the gap is that tissue samples cannot be completely severed from surrounding tissue without relying on tearing.

While the art associated with instruments which are intended to remove a tissue biopsy sample in a minimally invasive procedure is extensive, improvements are desired. In particular, a biopsy instrument which can be inserted minimally invasively to the site of the targeted lesion and remove a sufficiently large tissue sample is needed. In addition, such an instrument is needed which will facilitate the capture and cutting of the tissue sample once received within the instrument. Further, what is needed is an instrument which can severe the collected sample without unnecessarily and undesirably tearing the tissue as it is being severed.

### Summary of the Invention

The invention is a surgical instrument for excising a targeted tissue mass to be biopsied from adjacent bodily tissue of a surgical patient. The instrument comprises an elongated tube, a penetrating member, first and second tissue stops and a tissue cutter.

The elongated tube of the surgical instrument has proximal and distal ends. When the instrument is positioned to excise targeted tissue mass, the proximal end of the elongated tube is positioned externally of the surgical patient and the distal end of the elongated tube is positioned adjacent the targeted tissue mass.

The penetrating member is fixed to the distal end of the elongated tube. The penetrating member has a penetrating tip at a distal end of the penetrating member to facilitate the insertion of the distal end of the elongated tube adjacent the targeted tissue mass. A first tissue stop at a proximal end of the penetrating member extends radially outwardly from the elongated tube.

A second tissue stop extends radially outwardly from the elongated tube. The second tissue stop faces the first tissue stop and is disposed proximally of the first tissue stop. The first and second tissue stops define a tissue receiving chamber therebetween continuously about a 360° arc at the distal end of the elongated tube for receiving at least a portion of the targeted tissue mass within the tissue receiving chamber.

The tissue cutter is mounted concentrically relative to the elongated tube. The cutter has a distal cutting edge surface for cutting tissue. The cutter is movable relative to the penetrating member from a first non-cutting position where the cutting edge surface is spaced from the first tissue stop to a second tissue-cut position where the cutting edge surface is adjacent the first tissue stop.

When the penetrating member is positioned adjacent the targeted tissue mass to be excised for biopsy and the cutter is in the first non-cutting position, the targeted tissue mass or at least the portion thereof is received continuously about the distal end of the elongated tube within the tissue receiving chamber. When the cutter is subsequently moved from the first non-cutting position to the second tissue-cut position, the cutter encloses the tissue receiving chamber and the cutting edge surface of the cutter has excised the tissue received in the tissue receiving chamber from the adjacent bodily tissue.

The tissue receiving chamber defined between the first and second tissue stops for continuously receiving the targeted tissue mass about a 360° arc of the elongated tube enables the capture and excision of a significantly large tissue sample while maintaining all of the advantages of the minimally invasive approach to obtaining a biopsy. Unlike many conventional biopsy instruments, the instrument of this invention need not rely on a restricted and small lateral port embedded within the biopsy device for taking a confined tissue sample. Instead, the instrument of this invention takes full advantage of an entire radial surface of the distal end of an elongated tube to define a tissue receiving chamber to receive a sample size significantly greater than that achieved with conventional biopsy instruments containing confined lateral ports.

In addition, the surgical instrument of this invention does not rely on the passive capture of tissue into the tissue receiving chamber, but rather relies on the movement of the tissue cutter relative to the elongated tube from its first to second positions to actively sever the captured tissue. In this way, tearing of the tissue, and the undesirable consequences such tearing may cause, can be avoided or minimized.

In a particularly preferred embodiment of this invention, the surgical instrument further comprises a vacuum source in communication with the tissue receiving chamber for pulling and holding the targeted tissue mass or the portion thereof into the tissue receiving chamber and against the distal end of the elongated tube. The ability to hold tissue via vacuum will ensure that the tissue is not pushed out of the tissue receiving chamber when the tissue cutter is moved from its first to second positions. In so doing, a consistently large specimen can be obtained.

The instrument of this invention can be used whenever it is necessary or desirable to excise a targeted lesion from an adjacent tissue mass for the purpose of obtaining a biopsy of the excised tissue. In particular, this instrument is adapted for procuring a biopsy sample in a minimally invasive manner under local anesthesia. Advantageously, tissue is captured without tearing, therefore creating less trauma at the biopsy site during sampling, which in turn minimizes bleeding and also the formation of dense scar tissue that could be misinterpreted as a lesion.

### Brief Description of the Drawings

Figure 1 is an isometric view of the distal end of a preferred embodiment of the instrument of this invention. The instrument incorporates an obturator tip, an annular tissue cavity, a central suction tube, and an annular blade.
Figure 2 is a centerline section view of the instrument of Figure 1 prior to insertion into an expandable sleeve.
Figure 3 is a centerline section view of the instrument and expandable sleeve depicted in Figures 1 and 2 as the instrument is inserted therethrough.
Figure 4 is a centerline section view of the instrument shown in Figure 3 fully inserted into the expandable sleeve. The distal end of the sleeve is adjacent the targeted tissue mass.

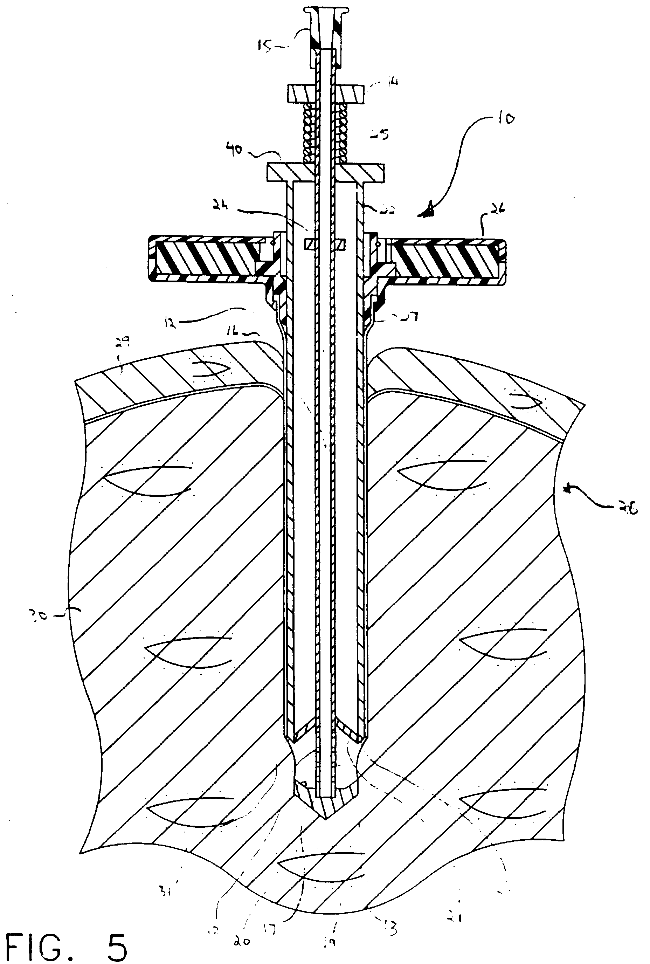

Figure 5 is a centerline section view of the instrument with its distal end extended into the targeted tissue mass.
Figure 6 is a centerline section view of the instrument with vacuum pressure applied to the tissue receiving chamber, thereby drawing in a sample mass of tissue.
Figure 7 is a centerline section view of the instrument illustrating the distal movement of the tissue cutter to excise the suspect tissue mass.

### Detailed Description of the Preferred Embodiment

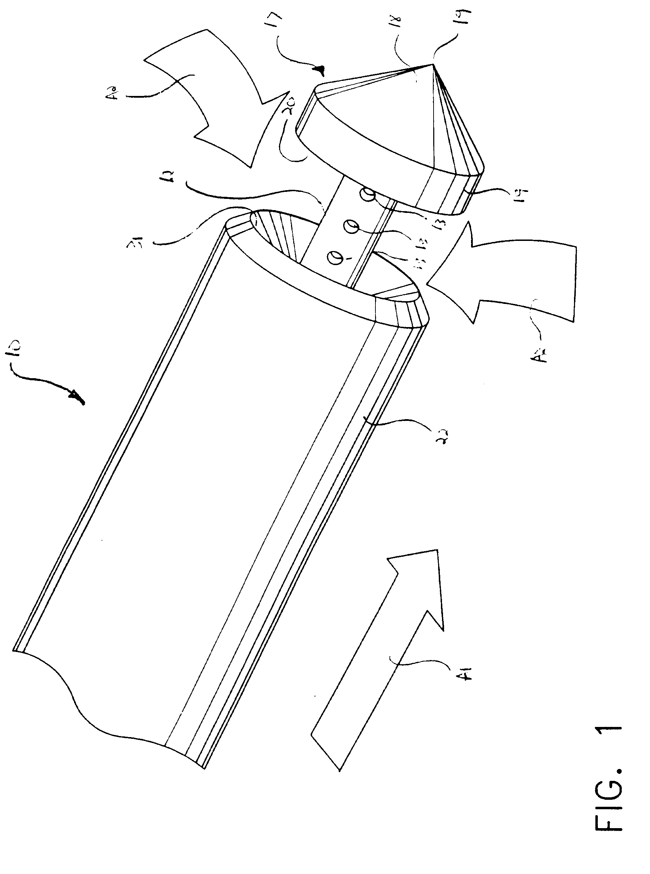

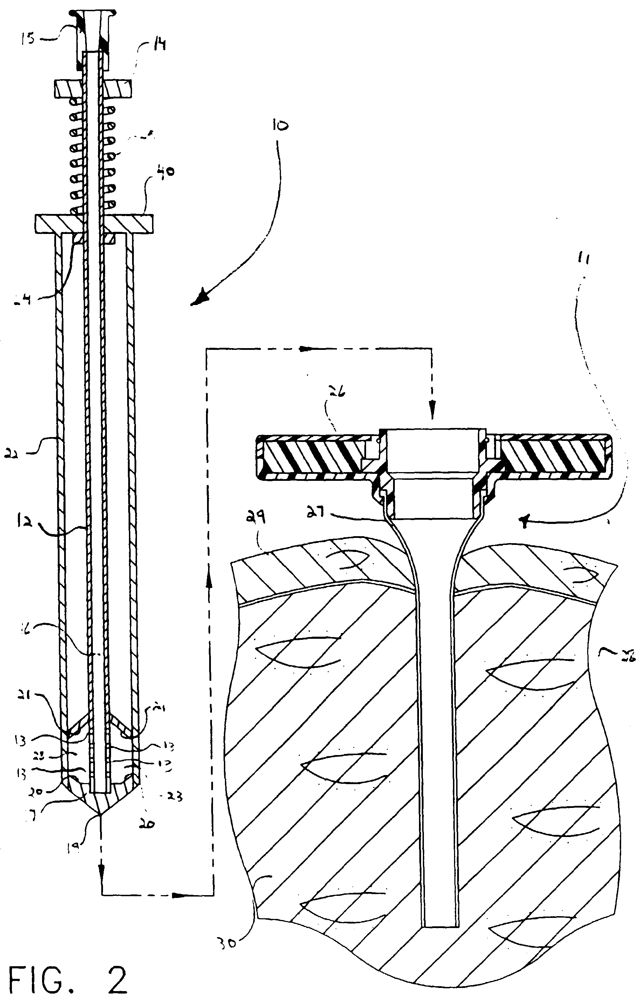

A perspective view of the preferred surgical instrument, specifically the tissue biopsy device 10 which is advantageously used with an expandable sleeve 11 to readily provide access to the targeted tissue mass, is shown in Figure 1. Figure 2 provides additional details in a sectional view of the tissue biopsy device and the expandable sleeve, as well as depicting the interrelationship between the device and the sleeve.

The biopsy device 10 has an elongated tube 12 with proximal and distal ends which extend from the proximal and distal ends of the device. At the distal end of the tube, there are a plurality of vacuum orifices 13 to pull a vacuum against the distal end of the tube. At the proximal end of the tube, there is a tube base 14 and a vacuum adapter 15 which is connected to a vacuum source for drawing the vacuum through the vacuum orifices and through the longitudinal channel 16 of the elongated tube (the vacuum source is not shown in Figures 1 or 2).

Affixed to the distal end of the tube of the surgical biopsy instrument is a penetrating member 17. The penetrating member has a sharp penetrating tip 18, and the penetrating tip has a conical configuration The penetrating member has a circular base 19 at its proximal end. The base is spaced from the apex 19 of the penetrating tip. The underside of the base of the penetrating member acts as a first tissue stop 20. The first tissue stop extends outwardly in the radial direction from the elongated tube. A second tissue stop 21 extends from the elongated tube and faces the first tissue stop (see Figure 2). Similarly to the first tissue stop, the second tissue stop extends outwardly in the radial direction from the elongated tube, and is located proximally of the first tissue stop.

The annular tissue cutter 22 of the biopsy device is mounted concentrically, about the elongated tube. It is movable longitudinally relative to the penetrating member affixed to the tube from a first non-cutting position to a second tissue-cut position. In Figure 2, the tissue cutter is shown in its first non-cutting position. In this position, the tissue cutter is located adjacent the base of the penetrating member, and the gap between the first and second tissue stops is enclosed.

The first and second tissue stops define a tissue receiving chamber 40 between the stops. This receiving chamber can receive tissue continuously about a 360° arc at the distal end of the tube where the vacuum orifices are placed. A greater amount of tissue can be loaded into the receiving chamber than what is capable if a conventional lateral tissue receiving port were used. Further, the outward radial dimension provided by the first and second tissue stops also increases the depth of tissue which can be loaded into the chamber.

The annular cutter 22 of the device has a cutter base 40 at a proximal end and a cutter stop 24 located adjacent to the cutter base. Interposed between the tube base and the cutter base is a cutter spring 25. The spring biases the annular cutter in the distal direction. Consequently, the spring biases the cutter toward its first non-cutting position.

The sleeve 11 which is used to receive the tissue biopsy device for providing the access portal to the target tissue mass is expandable. The sleeve is sized to receive the biopsy device and has a lengthwise dimension sufficient to extend to a position where the distal end of the sleeve is adjacent the targeted tissue mass. At a proximal end of the sleeve, there is a sleeve handle 26 for gripping and manipulating the sleeve. There is an expanded base region 27 transitioning between the sleeve handle 26 and the expandable sleeve 11. The sleeve is inserted into the tissue 28 to a location adjacent the target tissue mass using conventional means. When a breast biopsy is desired, the sleeve is inserted through the outer skin layer 29 of the breast and into the percutaneous breast tissue 30. When the sleeve is fully inserted, the sleeve handle 26 is located exteriorly of the outer skin layer 29 of the breast.

Importantly, the sleeve is expandable from a first original diameter to a greater, second expanded diameter to accomodate the diameter of the annular cutter of the biopsy device. The sleeve is originally inserted into the breast at its first original diameter, and then it is expanded to its second diameter when the biopsy device is inserted through the sleeve. The expanded second diameter is substantially the same as the diameter of the annular cutter of the device.

An expandable sleeve which can be used in the practice of this invention is described in U.S. Patent Nos. 3,789,852; 4,716,901; 4,899,729; 5,183,464 and 5,320,611

As depicted in Figure 1, the tissue biopsy device 10 is inserted in the direction depicted by directional arrow A1 to take advantage of the conical tip configuration of the penetrating member to facilitate ease of insertion. When the vacuum is pulled through the vacuum orifices against the distal end of the tube; tissue can be pulled into the tissue receiving chamber in the direction depicted by the directional arrows A2 as shown.

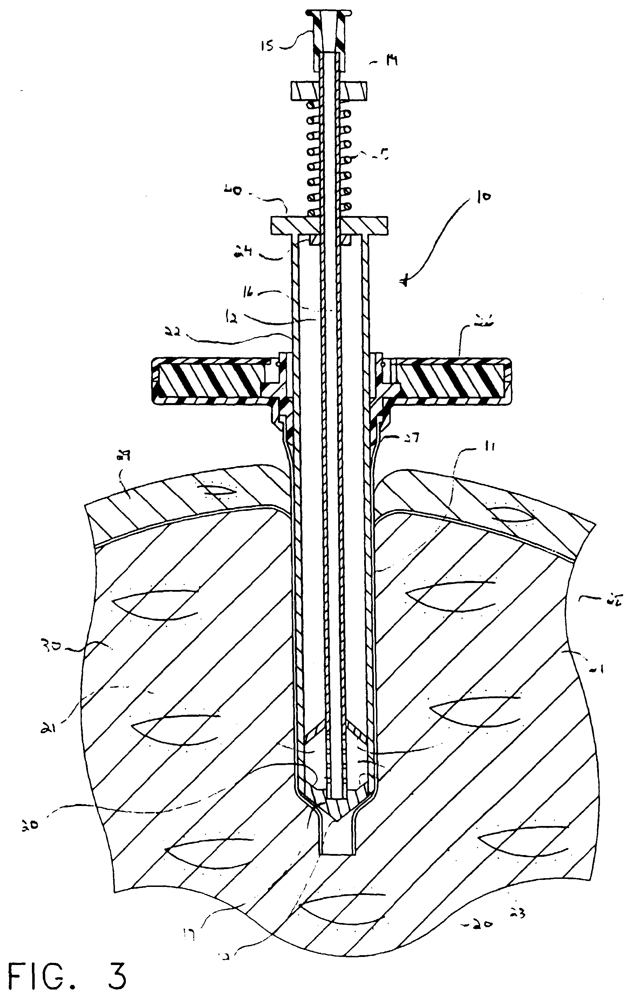

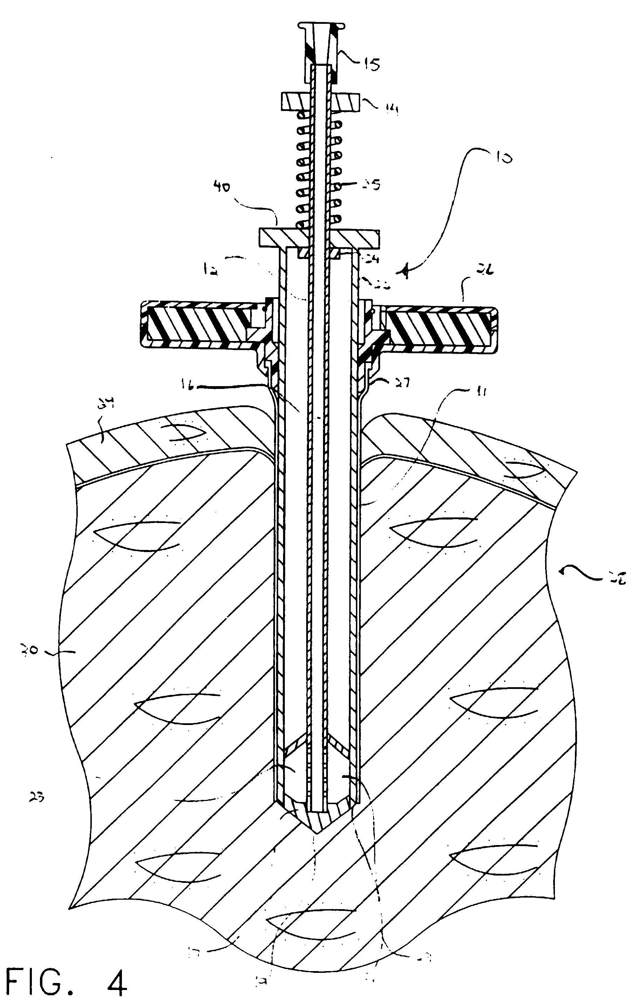

When the sleeve has been inserted into the tissue at its first original diameter, the tissue biopsy device can then be inserted through the sleeve, using the sleeve as an access portal to provide ease of insertion of the tissue biopsy device to a location where the tissue receiving chamber can be placed within the targeted tissue mass. As shown in Figure 3, when the biopsy instrument is inserted into the sleeve, the sleeve expands from its first original diameter to its second expanded diameter which corresponds to the diameter of the annular tissue cutter. In so doing, the tissue is dialated from the first original diameter to the second expanded diameter, causing less trauma to the patient than if an incision the size of the second expanded diameter were made. In Figure 4, the biopsy device is fully inserted, and the penetrating tip of the penetrating member affixed to the elongated tube extends from the distal end of the expanded sleeve. Additionally, when fully inserted, the proximal end of the tube of the biopsy device is positioned externally of the tissue, and the tube distal end is positioned percutaneously adjacent the targeted tissue mass.

Once the tissue biopsy instrument is inserted fully through the expanded sleeve, the annular cutter 22 is retracted from its first non-cutting position to its second tissue-cut position. A distal cutting edge surface 31 at the distal end of the annular cutter is exposed. This surface is located adjacent the first tissue stop when the cutter is retracted to its second tissue-cut position. The cutter is retracted by exerting an upper proximal force against the spring 25 (using the cutter base 40 as a grip to apply the force) to compress the spring. The cutter is then held in its second position. In the meantime, the elongated tube 12 of the tissue biopsy device, and the expandable sleeve 11, remain stationary relative to the movement of the annular tissue cutter 22.

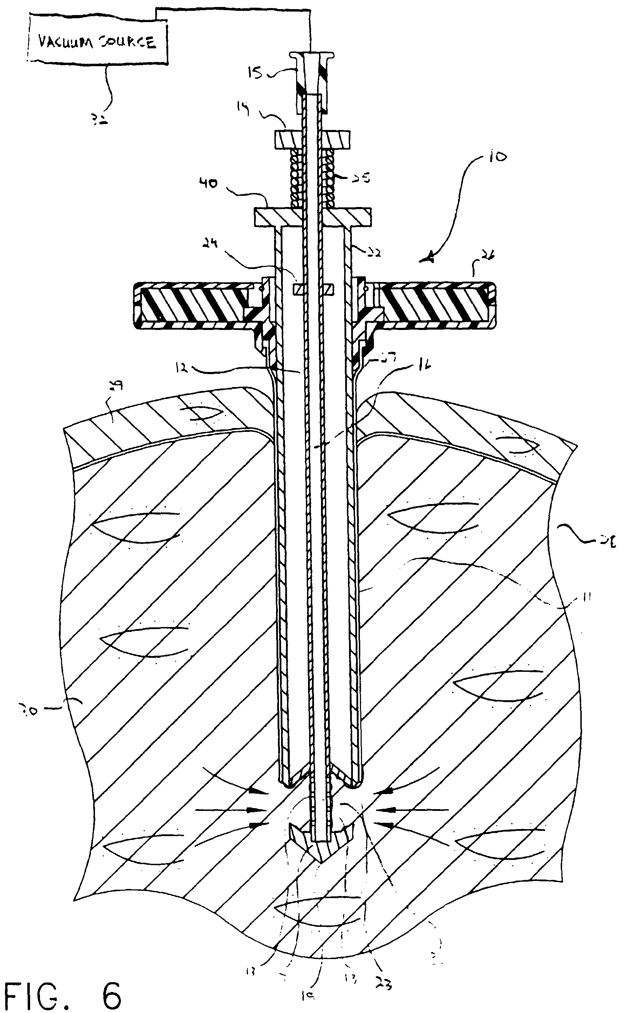

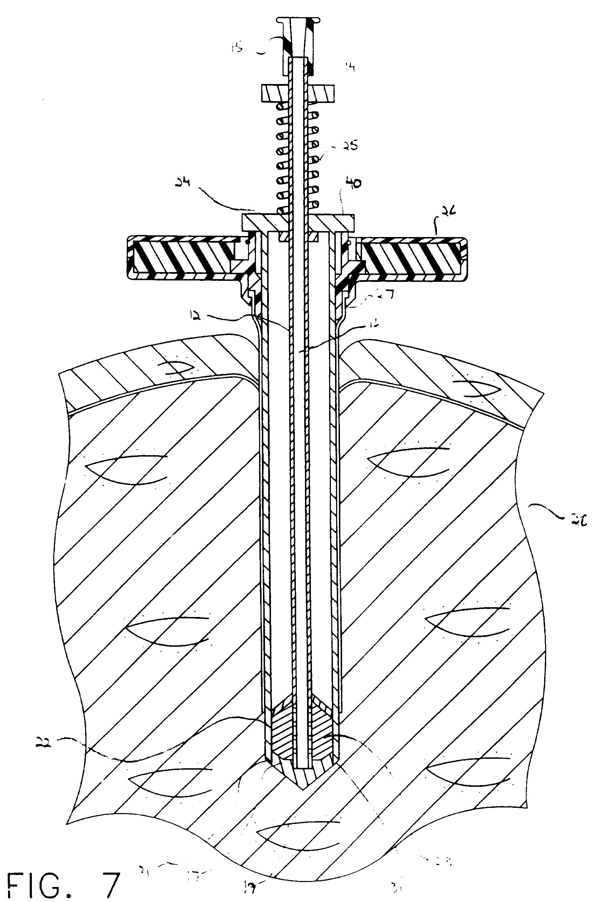

Referring now to Figure 6, with the annular cutter 22 in its retracted, second tissue-cut position, the vacuum adapter 15 is connected to a vacuum source 32 (see Figure 6), and vacuum is applied. When the vacuum is applied, at least a portion of the targeted tissue mass is pulled into the tissue receiving chamber and against the distal end of the elongated tube as illustrated by the directional arrows depicted in Figure 6. The vacuum is drawn through the vacuum orifices and into the longitudinal channel 16 of the elongated tube 12. Once the tissue is loaded into the tissue receiving chamber 23 as a consequence of the application of vacuum, the annular cutter can be released from its second position. When it is released, the distal cutting edge surface 31 of the annular cutter excises the tissue loaded into the chamber as the cutter slides longitudinally from its second tissue-cut position to its first non-cutting position. When the annular cutter returns to its first non-cutting position, the desired portion of the targeted tissue mass is fully enclosed within the continuous tissue receiving chamber of the device as depicted in Figure 7. From this point, the device can be removed from the sleeve, and the sample of tissue in the chamber can be extracted from the chamber using conventional techniques.

Although this invention has been described in connection with its most preferred embodiment, this particular embodiment is provided by way of example, and should not be used to limit the scope of the claimed invention. The scope of the invention is defined by the claims which follow.

## Claims

1. A surgical instrument for excising a targeted tissue mass to be biopsied from adjacent bodily tissue of a surgical patient, said instrument comprising:
a) an elongated tube having proximal and distal ends, and when said instrument is positioned to excise the targeted tissue mass, the proximal end of said elongated tube is positioned externally of the surgical patient and the distal end of said elongated tube is positioned adjacent the targeted tissue mass;
b) a penetrating member affixed to the distal end of said elongated tube, said penetrating member having a penetrating tip at a distal end of said penetrating member to facilitate the insertion of the distal end of said elongated tube adjacent the targeted tissue mass, and a first tissue stop at a proximal end of said penetrating member extending radially outwardly from said elongated tube;
c) a second tissue stop extending radially outwardly from said elongated tube, said second tissue stop facing said first tissue stop and disposed proximally of said first tissue stop, said first and second tissue stops defining a tissue receiving chamber therebetween continuously about a 360 degree arc at the distal end of said elongated tube for receiving at least a portion of the targeted tissue mass therein; and
d) a tissue cutter mounted concentrically relative to said elongated tube, said cutter having a distal cutting edge surface for cutting tissue, said cutter movable relative to said penetrating member from a first non-cutting position wherein said cutting edge surface is spaced from said first tissue stop to a second tissue-cut position wherein said cutting edge surface is adjacent said first tissue stop;
wherein when said penetrating member is positioned adjacent the targeted tissue mass to be excised for biopsy and said cutter is in said first non-cutting position, the targeted tissue mass or at least the portion thereof is received continuously about the distal end of said elongated tube within the tissue receiving chamber, and when said cutter is subsequently moved from said first non-cutting position to said second tissue-cut position, said cutter encloses said tissue receiving chamber and said cutting edge surface of said cutter has excised the tissue received in said tissue receiving chamber from the adjacent bodily tissue.

2. The instrument of Claim 1 further comprising a vacuum source in communication with said tissue receiving chamber adjacent the distal end of said elongated tube for pulling the targeted tissue mass or the portion thereof into said tissue receiving chamber and against the distal end of said elongated tube.

3. The instrument of Claim 1 wherein said tissue cutter has a tubular configuration, and said elongated tube is mounted within said tissue cutter for relative sliding movement between said tissue cutter and said elongated tube.
